Europäisches Patentamt

**European Patent Office**

**Office européen des brevets**

(19)

(11) Veröffentlichungsnummer: **0 029 245**
**A1**

(12) # EUROPÄISCHE PATENTANMELDUNG

(21) Anmeldenummer: **80107127.5**

(22) Anmeldetag: **17.11.80**

(51) Int. Cl.³: **A 61 N 1/04**, A 61 B 5/04

(30) Priorität: 20.11.79 DE 7932779 U
20.11.79 DE 7932816 U

(43) Veröffentlichungstag der Anmeldung: **27.05.81**
**Patentblatt 81/21**

(84) Benannte Vertragsstaaten: **AT BE CH DE FR GB IT LI NL
SE**

(71) Anmelder: **SIEMENS AKTIENGESELLSCHAFT Berlin
und München, Postfach 22 02 61,
D-8000 München 22 (DE)**

(72) Erfinder: **Naser, Georg, Karlstrasse 10, D-8502 Zirndorf
(DE)**
Erfinder: **Strahwald, Franz, Mühlgraben 6,
D-8553 Ebermannstadt (DE)**
Erfinder: **Szehl, Erich, Dipl.-Phys., Am Eichengarten 11,
D-8521 Buckenhof (DE)**

(54) **Anordnung zur Zuführung oder Abnahme von elektrischen Signalen.**

(57) Anordnungen zur Zuführung oder Abnahme von elektrischen Signalen können insbesonders als Elektrodenanordnung mit Mehrfachelektrode für die Interferenzstromtherapie verwendet werden. Solche Elektrodenanordnungen weisen üblicherweise ein Gehäuse als Träger einzelner Kontaktteile zum Aufsetzen am Applikationsort auf, wobei zwischen Elektrodenkontaktteilen und Applikationsort ein Träger für ein Kontaktierungsmittel legbar ist; vom Gehäuse führen Anschlussleitungen zum Betriebsgerät oder dgl. Ziel der Erfindung ist es, eine möglichst einfache Applikation der Elektrodenanordnung zu gewährleisten. Erfindungsgemäss ist das Gehäuse ein flächenhaftes, flexibles Formteil (2, 10) aus elektrisch isolierendem Kunststoff, in das als Elektrodenkontaktteile (3 bis 5, 11 bis 13) flexible, elektrisch leitfähige Kunststoffteile glatt eingebettet sind. Im Rahmen der Erfindung ist der Träger für das Kontaktierungsmittel ein Vliesstoffteil (42), das zusammen mit einer Abdeckung (43) eine Tasche (41) für die Mehrfachelektrode (1, 9) bildet. Es ergibt sich aufgrund weitgehend gleicher Flexibilität eine gute Anpassung der gesamten Anordnung an wechselnde Konturen des Applikationsortes an einen Patientenkörper.

0029245

SIEMENS AKTIENGESELLSCHAFT       Unser Zeichen
Berlin und München               VPA 79 P 5116 EUR

**Anordnung zur Zuführung oder Abnahme von elektrischen Signalen**

Die Erfindung bezieht sich auf eine Anordnung zur Zuführung oder Abnahme von elektrischen Signalen, insbesondere Elektrodenanordnung für die Interferenzstromtherapie, mit einer Mehrfachelektrode, bestehend aus einem Gehäuse als Träger von Elektrodenkontaktteilen zum Aufsetzen am Applikationsort, wobei zwischen den Elektrodenkontaktteilen und Applikationsort ein Träger für ein Kontaktierungsmittel legbar ist, sowie mit Anschlußleitungen an den Kontaktteilen, die vom Gehäuse in einer gemeinsamen Leitung zu einem Betriebsgerät od. dgl. führen,

Anordnungen dieser Art ermöglichen die Zuführung elektrischer Signale zu einem Patientenkörper, beispielsweise von Reizströmen bei der Reizstrombehandlung. Ebensogut können solche Anordnungen jedoch auch zur Abnahme bioelektrischer Signale, wie EKG od.dgl., von einem Körper eingesetzt werden. Wesentlich ist doch in jedem Fall, daß bei Applikation die Elektrodenkontaktteile gut kontaktieren, so daß Signale zum Patientenkörper möglichst ströungsfrei zugeführt oder vom Patientenkörper über die Elektroden störungsfrei abgenommen werden können. Dazu sind im allgemeinen am Applikationsort zwischen Elektroden und Haut zusätzliche Kontaktierungsmittel notwendig. Solche Kontaktierungsmittel können im einfachsten Fall für Kontaktflüssigkeit saugfähige Schwammkörper sein, die sich aufgrund ihrer Weichheit gut an das zu behandelnde Körperteil anschmiegen. Als geeignet haben sich hierfür auch dünne saugfähige

Wht 5 Rl / 14. 11.1980

Papiere, Vliesstoffe od.dgl. erwiesen, die zur Herstellung des elektrischen Kontaktes lediglich mit Wasser angefeuchtet werden müssen.

Besonders bei der mehrkreisigen Reizstrombehandlung ist es wichtig, die einzelnen Reizströme über die Elektroden gezielt an geometrisch vorbestimmten Orten des Patientenkörpers einzuprägen. Neben der separaten Applikation von Einzelelektroden werden dazu auch sog. Elektroden-Sets mit mehreren Kontaktteilen benutzt, bei denen bereits die erforderliche Anzahl der Kontaktteile in vorgegebener Geometrie miteinander verbunden ist. Solche Elektroden-Sets werden in Einsätze von kompakten Schwamm-Material aus Viskose od.dgl. eingefügt, wobei der gesamte Schwammeinsatz gleichermaßen als Träger der Kontaktflüssigkeit und als Halterung für die Einzelelektrodenkontaktteile dient. Der weiche Schwammeinsatz soll dabei aufgrund seiner leichten Verformbarkeit die notwendige Anpassung an die Körperkontur gewährleisten. Dieses hat für den Routinebetrieb Vorteile, da lediglich jeweils ein oder zwei vorbereitete Schwammeinsätze mit den Elektroden-Sets am zu behandelnden Körperteil appliziert werden müssen.

Speziell bei Verwendung dreier Stromkreise zur Erzielung eines stereodynamischen Interferenzeffektes der Reizströme sind auch bereits Elektrodenanordnungen in Sternform, aber auch in Reihenform bekannt. Zur Befestigung dieser speziellen Elektroden-Sets am Patientenkörper wird ebenfalls wieder eine Anpassung an die Körperkontur gefordert. Bisher werden dazu bewegliche Drahtgewebeeinsätze als Elektrodenkontaktteile in Moosgummiträger eingesetzt, wobei hier die gesamte Kontaktfläche mit einem Schwammtuch oder Papier abgedeckt ist. Nachteil derartiger Anordnungen war bisher, daß meist nicht die gewünschte Weichheit und Flexibilität erreicht wurde. Im Einzelfall konnte auch durch die weitgehend offenliegenden Drahtgewebe bei brechendem oder sich lösendem Draht und Durchstoßen von Drahtenden die Gefahr von Verbrennungen nicht ausgeschlossen werden.

Aufgabe der Erfindung ist es daher, eine Anordnung der eingangs genannten Art zu schaffen, die hinsichtlich ihrer Anwendungseigenschaften weiter verbessert ist. Insbesondere soll die Gefahr der Verbrennungen durch freie Drahtenden u.dgl. vollständig beseitigt werden. Dabei soll die gesamte Anordnung einschließlich des Kontaktmittels einfach applizierbar und auch über längere Zeiten benutzbar sein.

Die Aufgabe wird erfindungsgemäß dadurch gelöst, daß das Gehäuse ein flächenhaftes flexibles Formteil aus elektrisch isolierendem Kunststoff ist, in das als Elektrodenkontaktteile flexible, elektrisch leitfähige Kunststoffteile glatt eingebettet sind.

Die erfindungsgemäße Anordnung hat den Vorteil einer weitgehend gleichbleibenden Flexibilität. Isolierendes Formteil aus Kunststoff und Kontaktteile bilden durch Vulkanisieren zur Applikationsfläche hin eine allseitig geschlossene, glatte Oberfläche. Dadurch ist eine einfache und sichere Reinigung und Desinfektion der gesamten Elektrodenanordnung möglich.

Vorzugsweise ist das leitende Kontaktteil zur besseren Stromverteilung rückseitig mit einem Drahtgewebe verbunden. Bei Herstellung einer solchen Elektrodenanordnung wird ein solches Drahtgewebe zunächst an oder in den leitfähigen Kunststoff vulkanisiert, bevor der-

0029245

artige Einsätze in das Formteil aus nichtleitendem Kunststoff eingesetzt und noch einmal durch Wärmebehandlung innig miteinander verbunden werden. Dabei können die Kontakteinsätze leicht gegenüber dem Flachteil herausragen, so daß sie mit Sicherheit immer an der Körperoberfläche anliegen. Dazu ist ein umlaufender Keilwulst um das Kontaktteil im isolierenden Formteil gebildet, der zur besseren Verankerung im Formteil dient.

In Ausbildung als Dreifachelektrode sind die Kontaktteile vorzugsweise in je um 120$^o$ versetzten Lappen angeordnet. Es wird so eine sternförmige Elektrode gebildet. Die Kontaktteile können aber auch in bandförmiger Flächenform hintereinander angeordnet sein. Form und Größe der gesamten Elektrodenanordnung sind zur Verwendung an verschiedenen Körperteilen für den betreffenden Bedarf angepaßt.

Die Elektrodenanordnungen können einen integrierten Leitungsanschluß für ein Betriebskabel mit Betriebsstecker aufweisen. Es kann jedoch auch ein Steckanschluß direkt an der Elektrodenanordnung vorgesehen sein, der insbesondere zum schnellen Austausch der Elektroden-Sets dient.

Im Rahmen der Erfindung ist der Träger für das Kontaktmitel ein Vliesstoffteil, dessen Kontur geringfügig größer als der äußere Umfang des Formteiles der Elektrodenanordnung ist, wobei das Vliesstoffteil auf seiner der Applikationsfläche abgewandten Seite eine Abdeckung aus Isoliermaterial aufweist, so daß die flexible Mehrfachelektrode zwischen Vliesstoffteil und Abdeckung einschiebbar ist.

Dabei ist der Vliesstoff vorzugsweise mit der Abdeckung bis auf eine Kante am Umfang zusammengeheftet. Es wird also eine Elektrodentasche gebildet; gleichzeitig wird durch Verwendung einer Folie aus Polyvinylchlorid oder dgl. als Abdeckung erreicht, daß ein Austrocknen des Fliesstoffes weitgehend verhindert wird. Die Auswirkungen von Temperatur- und Feuchtigkeitsunterschieden der Umgebungsluft werden so eliminiert. Insgesamt wird auch über längere Behandlungszeiträume die Feuchtigkeit im Vlies gehalten und somit fortwährend eine gute elektrische Kontaktierung gewährleistet.

Weitere Vorteile und Einzelheiten der Erfindung ergeben sich aus der nachfolgenden Figurenbeschreibung von Ausführungsbeispielen anhand der Zeichnung in Verbindung mit den übrigen Unteransprüchen. Es zeigen:

Fig. 1 die Ansicht auf die Applikationsseite einer erfindungsgemäß ausgebildeten Sternelektrode,

Fig. 2 eine entsprechende Ansicht auf eine bandförmig ausgestaltete Dreifachelektrode,

Fig. 3 die Rückansicht auf den Elektrodenstern nach Fig. 1 in teilweise aufgeschnittenem Zustand,

Fig. 4 einen Schnitt durch Fig. 3 längs der Linie IV-IV,

Fig. 5 einen Querschnitt durch ein Kupplungsteil als Elektrodenanschluß,

Fig. 6 einen als Elektrodentasche ausgebildeten Träger für Kontaktmittel in der Draufsicht,

Fig. 7 einen Schnitt durch Fig. 6 längs der Linie VII-VII,

Fig. 8 eine Stoffbahn mit doppelter Stanzkontur zum Herstellen eines Vliesteiles mit beidseitig glatten Oberflächen und

Fig. 9 die Elektrodentasche nach Fig. 6 mit darin eingelegter Sternenelektrode nach Fig. 1.

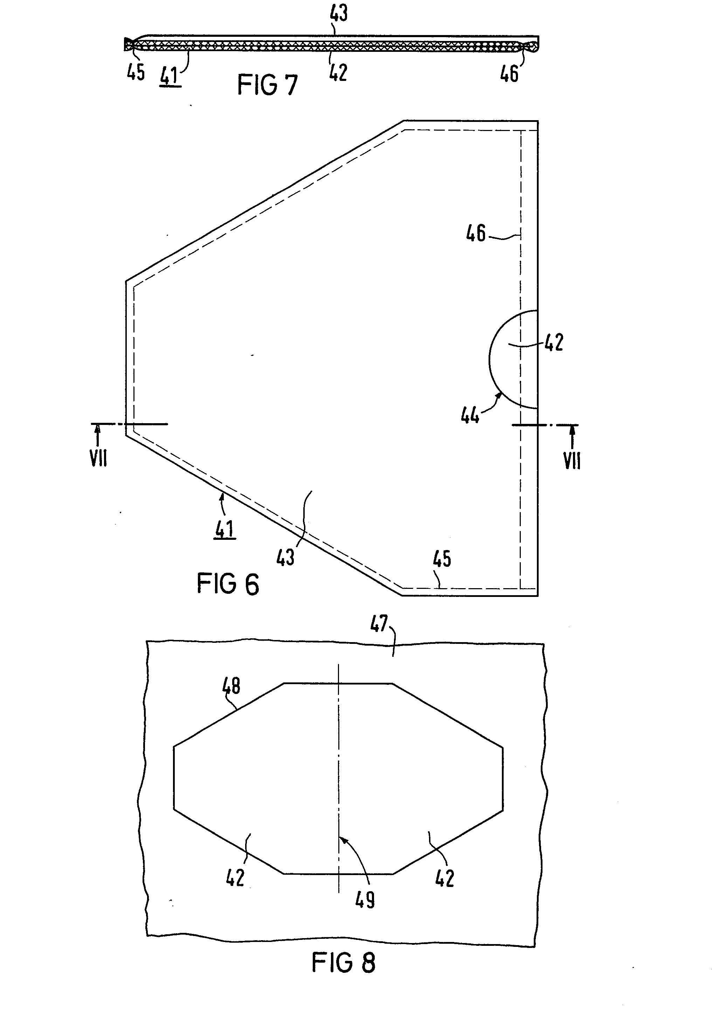

Die Figuren sind teilweise im verkleinerten und teilweise im natürlichen Maßstab eines Ausführungsbeispiels dargestellt; speziell Fig. 5 ist demgegenüber vergrößert dargestellt. Identische Teile der Figuren sind mit den gleichen Bezugszeichen versehen.

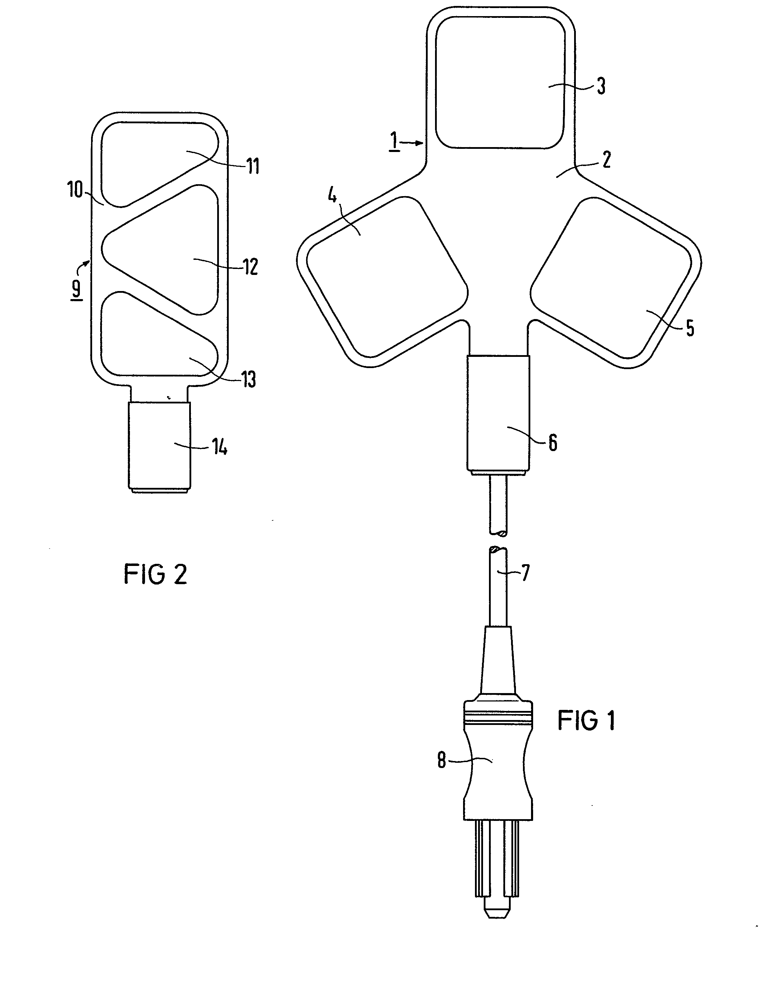

In der Fig. 1 ist mit 1 eine Sternelektrode mit einem Formteil 2 aus elektrisch isolierendem Kunststoff bezeichnet, das aus drei quadratischen Lappen, die jeweils um 120$^o$ versetzt sind, sternförmig zusammengesetzt ist. Die Kanten dieser Lappen sind leicht abgerundet, wobei in jedem dieser Lappen ein quadratisches Kontaktteil 3 bis 5 eingefügt ist. Zwischen zwei der drei Kontaktteile 3 bis 5 ist am Formteil 2 ein Kupplungsteil 6 als Leitungsanschluß angebracht, dessen Aufbau weiter unten noch erläutert wird. Über ein Betriebskabel 7 mit Betriebsstecker 8 ist die Elektrodenanordnung an ein (nicht dargestelltes) Reizstrombetriebsgerät anschließbar.

In der Fig. 2 ist eine Elektrodenanordnung als Bandelektrode 9 mit einem Formteil 10 aus elektrisch isolierendem Kunststoff ausgebildet, in das hintereinander etwa dreieckförmige Kontaktteile 11 bis 13 derart eingefügt sind, daß die gegeneinander versetzten Flächenschwerpunkte der Einzeldreiecke wiederum eine Dreiecksform bilden. Ein Betriebskabelanschluß 14 ist hier als Steckkupplung ausgebildet.

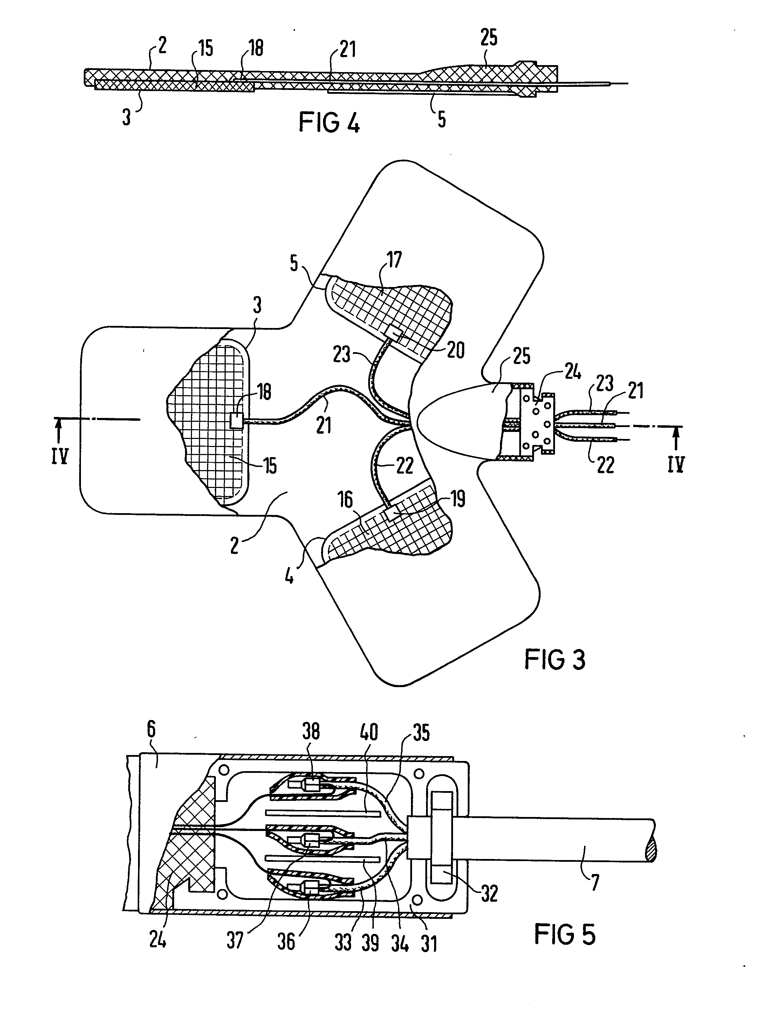

In der Fig. 3 ist die Rückseite des Kunststofflachteiles nach Fig. 1 wieder mit 2 bezeichnet. Im halbaufgeschnittenen Zustand des Formteiles 2 sind die Rückseiten der Kontaktteile 3 bis 5 nach Fig. 1 sichtbar. Es sind jeweils Drahtnetze 15 bis 17 auf den Rückseiten aufgebracht. Diese dienen im wesentlichen zur besseren Stromverteilung über die gesamten Flächen der Leitkontakte. Die Drahtnetze 15 bis 17 weisen jeweils Anschlüsse 18 bis 20 auf, von denen elektrische Leitungen 21 bis 23 in Schleifen zu einem Kupplungsteil 24 als Anschluß geführt sind. Im Bereich des Anschlusses ist zur besseren Stabilität das Kunststofformteil verdickt ausgebildet.

Dieser Aufbau wird auch im Schnittbild nach Fig. 4 deutlich.

Zur Herstellung einer solchen Elektrodenanordnung wird zunächst in einer Form ein Drahtgeflecht mit Anschlußleitung mit einem leitfähigen Silikongummi in vorgegebener Flächenform, die etwas größer als das Drahtnetz ist, durch Wärmebehandlung verbunden, so daß sich einseitig eine vollständig glatte Oberfläche ergibt. Die so vorbereiteten leitfähigen Einsätze werden dann in eine Form eingepaßt und mit dem elektrisch nicht leitenden Kunststoff durch Erwärmung verbunden. Durch Vulkanisieren ergibt sich zwischen dem leitfähigen Kunststoff und isolierendem Kunststoff an den Grenzflächen eine vollständig dichte Verbindung. Dies ist insbesondere deshalb von Vorteil, da so die Elektrodenanordnung nach Gebrauch als Gesamtes gereinigt und sterilisiert werden kann. Die Anschlußleitungen 21 bis 23 der Kontaktteile 3 bis 5 sind in Teflonummantelungen bei der Wärmebehandlung in den nichtleitenden Kunststoff schleifenförmig derart eingebettet, daß die Flexibilität der gesamten Elektrodenanordnung nicht beeinträchtigt und die Leitungen selbst nicht auf Zug belastet werden. Die leitfähigen Kontaktteile weisen zweckmäßigerweise eine umlaufende Keilwulst auf, wodurch beim Vulkanisieren der Kontaktteile in das isolierende Trägerteil die mechanische Verankerung in der Isolierplatte weiterverbessert wird.

Da das eigentliche Betriebskabel 7 zum Anschluß an ein Betriebsgerät zweckmäßigerweise aus Lahnleitern besteht, ist es notwendig, den Leitungsanschluß zum Formteil 2 als Kupplungsteil auszubilden. Dies kann entweder als Crimp-Verbindung, oder als Stecker ausgebildet sein. In der Fig. 5 ist ein solches Kupplungsteil als feste Verbindung dargestellt. Es besteht aus zwei Halbschalen 30 und 31, die an der einen Seite auf den Anschluß 24 nach Fig. 3 aufsetzbar sind und auf der anderen Seite eine Aussparung 32 für eine Zugentlastung 32 des Betriebskabels aufweisen. Die Einzelleitungen 33 bis 35 des Betriebskabels sind in diesen so gebildeten Gehäuse über Crimp-Anschlüsse 36 bis 38 mit den Leitungen 21 bis 23 verbunden. Zwischen den einzelnen Crimp-Anschlüssen 36 bis 38 sind Stege 39 und 40 als Isolationsteile angeordnet.

Die oben beispielhaft für eine Sternelektrode beschriebene Ausbildung und Ausfertigung der erfindungsgemäßen Elektrodenanordnung kann auf die Zusammenfassung von Einzelelektroden beliebiger Anzahl und beliebiger Anordnung übertragen werden. Es empfiehlt sich dabei Flächenform und Größe für die verschiedenen Anwendungszwecke anzupassen wobei
solche Anordnungen gegebenenfalls über das Kupplungsteil leicht ausgetauscht werden können.

In den Fig. 6 und 7 und 9 ist mit 41 eine Elektrodentasche bezeichnet. Sie besteht aus einer Grundfläche
42 aus Wirrfaservliesstoff, auf der eine Folie 43 aus
Polyvinylchlorid oder ähnlichem festgeheftet ist. Speziell zur Aufnahme einer als sog. Sternelektrode nach
Fig. 1 und 4 ausgebildeten Dreifachelektrode ist die
Grundfläche 42 etwa dreiecksförmig und wenigstens
gleichschenklig ausgebildet, wobei die Ecken dieses
gleichschenkligen Dreiecks derart beschnitten sind, daß
Symmetrie zu der auf der Grundlinie verlaufenden Mittelsenkrechten vorliegt. Es ergibt sich so insgesamt die
dargestellte sechseckige Grundfläche mit je zwei paarweise zueinander parallelen Kanten. Die beiden Dreieckskanten können wahlweise auch konkav ausgebildet
sein. Die Abdeckfolie 43 weist zur Grundkante der Tasche
41 hin eine halbkreisförmige Ausnehmung 44 auf.

Als Vliesstoffmaterial wird ein Wirrfaservliesstoff auf
Zellwollbasis mit Polyolefinfasern verwendet. Ein derartiges Material ist im Handel unter dem Namen "VILEDON
T 1557" speziell für medizinische Anwendungen, wie Wundabdeckungen od.dgl., bekannt. Es zeichnet sich insbesondere durch gute Hautverträglichkeit aus und ist auch
über längere Zeiträume hygienisch einwandfrei zu verwenden. Sowohl im trockenen als auch im feuchten Zustand ist dieses Material weich und schmiegsam, so daß
es an beliebig geformte Körperteile anlegbar ist; zwecks
elektrischer Kontaktgabe ist es gut für eine hinreichende Flüssigkeitsmenge saugfähig.

Im allgemeinen weist der im Handel befindliche Wirrfaservliesstoff bereits wenigstens eine vollständig glatte Seite auf. Da dieses Material relativ dünn ist, hat es sich als zweckmäßig erwiesen, aus der Rohstoffbahn ein Teil mit doppelter Grundfläche auszustanzen, das spiegelsymmetrisch zu einer Mittelebene ausgebildet ist. Längs dieser Symmetrieebene wird das Stanzteil dann lediglich gefaltet, wobei zweckmäßigerweise die Oberflächen bereits die vollständig glatt ausgebildeten Flächen bilden. In der Fig. 8 ist ein solches Stanzteil 48 aus einer Stoffbahn 47 dargestellt.

Nach Zusammenfalten wird auf die Grundfläche nach Fig. 6 die entsprechend ausgebildete Folie aufgelegt und mittels einer Steppnaht 45 angeheftet. Längs der Naht 46 wird lediglich das Doppelteil verbunden. An dieser Grundlinie ist also die vom Fliesstoffteil 42 und Folie 43 gebildete Tasche offen. Eine Elektrodenanordnung kann hier leicht eingeschoben werden.

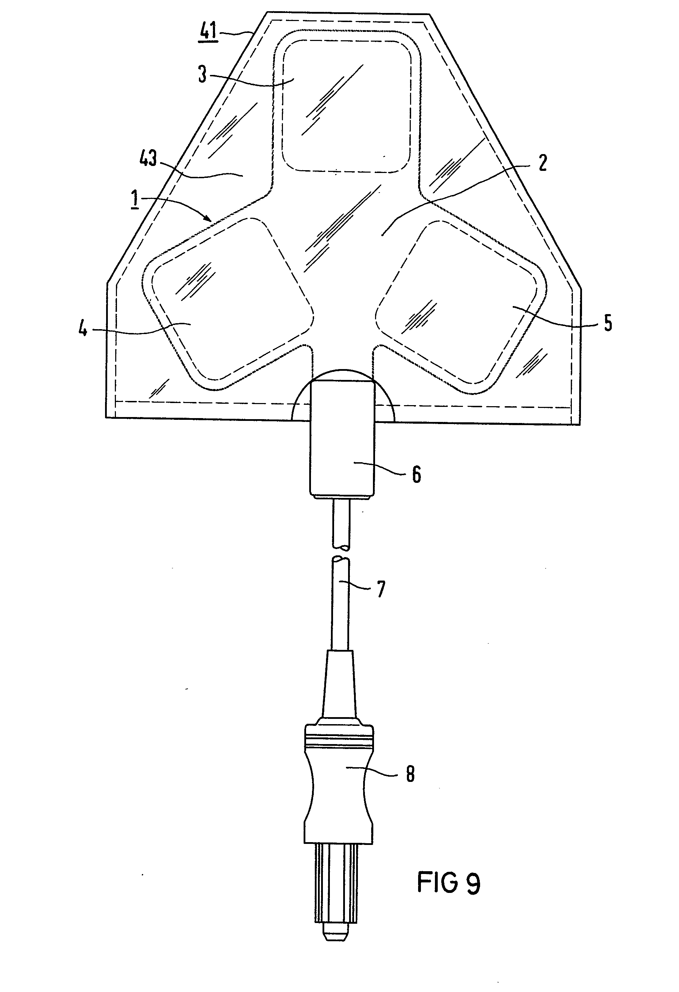

In der Fig. 9 ist  in eine Elektrodentasche 41 nach Fig. 6 eine als Sternelektrode ausgebildete Dreifachelektrode 1 eingelegt, die anhand der Figuren 1, 3 und 4 beschrieben wurde. Ist wie in Fig. 9 eine solche Elektrodenanordnung korrekt eingeschoben, so sitzt deren Anschluß  6 in der halbkreisförmigen Ausnehmung 44 der Abdeckung 43. Durch Verwendung eines durchsichtigen Folienmaterials als Abdeckung 43 kann bei Applikation ständig  der korrekte Sitz der Elektrodenanordnung überprüft werden.

Wie bereits erwähnt, ist speziell Wirrfaservliesstoff wegen seiner Weichheit und Schmiegsamkeit besonders gut für die erfindungsgemäße Vorrichtung geeignet. Durch die Folienabdeckung an der der Applikationsfläche abgewandten Seite wird bei Gebrauch die Verdampfung von Kontaktflüssigkeit zur Umgebung weit-

0029245

gehend verhindert und neben der Feuchtigkeitsisolierung auch eine elektrische Isolation gewährleistet. Aufgrund der Hautfeuchtigkeit des Patienten und der weitgehend dichten Abdeckung zur Umgebung ergeben sich so am Applikationsort des Patientenkörpers auch über längere Behandlungszeiten ideale Kontaktierungsverhältnisse.

Die Elektrodentasche aus Vliesstoff und isolierender Abdeckung wurde speziell für eine Sternelektrode beschrieben. Bei anderen Elektrodenanordnungen, beispielsweise einer Bandelektrode nach Fig. 2, ist die Größe der Tasche ganz entsprechend auf Größe und Form des betreffenden Elektroden-Sets abgestimmt und zwar gerade so in geringfügig größerer Ausbildung, daß das Elektroden-Set zwar bequem eingeschoben werden kann, dort aber gut fixiert ist. Solche Bandelektroden-Sets können einschließlich der zugehörigen Tasche beispielsweise Arme oder Beine eines Patienten umschließen.

Für die Applikation der Elektrodenanordnung wird der Vliesstoff angefeuchtet, wodurch so der Übergangswiderstand der Elektrodenkontaktteile zur Haut herabgesetzt wird. Unerwünschte Querströme zwischen den Elektrodenfeldern sind bei Verwendung von Vliesstoffen geringer Dicke vernachlässigbar klein.

Insgesamt ergibt sich so aufgrund weitgehend gleicher Flexibilität, Weichheit und Anschmiegsamkeit aller verwendeten Werkstoffe eine unproblematische Anlage und Fixierung der erfindungsgemäßen Anordnung am Patientenkörper. Natürlich kann eine derartige erfindungsgemäße Anordnung außer für die Zuführung von Reizströmen auch zur Abnahme bioelektrischer Signale vom Patientenkörper, wie EKG od.dgl., verwendet werden.


9 Figuren
13 Patentansprüche

Patentansprüche

1. Anordnung zur Zuführung oder Abnahme von elektrischen Signalen, insbesondere Elektrodenanordnung für die Interferenzstromtherapie, mit einer Mehrfachelektrode, bestehend aus einem Gehäuse als Träger von Elektrodenkontaktteilen zum Aufsetzen am Applikationsort, wobei zwischen den Elektrodenkontaktteilen und Applikationsort ein Träger für ein Kontaktierungsmittel legbar ist, sowie mit Anschlußleitungen an den Kontaktteilen, die vom Gehäuse in einer gemeinsamen Leitung zu einem Betriebsgerät od. dgl. führen, d a d u r c h  g e k e n n z e i c h n e t ,  daß das Gehäuse ein flächenhaftes flexibles Formteil (2, 10) aus elektrisch isolierendem Kunststoff ist, in das als Elektrodenkontaktteile (3 bis 5, 11 bis 13) flexible, elektrisch leitfähige Kunststoffteile glatt eingebettet sind.

2. Elektrodenanordnung nach Anspruch 1, d a d u r c h g e k e n n z e i c h n e t ,  daß in oder an die Rückseite der elektrisch leitfähigen Kunststoffteile (3 bis 5, 11 bis 13) ein Drahtgewebe (15 bis 17) eingebetten, vorzugsweise vulkanisiert, ist.

3. Elektrodenanordnung nach Anspruch 1, d a d u r c h g e k e n n z e i c h n e t ,  daß das Formteil (2, 10) aus isolierendem Kunststoff und die Kontaktteile (3 bis 5, 11 bis 13) aus leitendem Kunststoff durch Vulkanisieren dicht verbunden sind.

4. Elektrodenanordnung nach Anspruch 1, d a d u r c h g e k e n n z e i c h n e t ,  daß die Kontaktteile (3 bis 5, 11 bis 13) aus dem Formteil (2, 10) herausragen.

0029245

5. Elektrodenanordnung nach Anspruch 1, d a d u r c h
g e k e n n z e i c h n e t , daß in Ausbildung als
Dreifachelektrode (1) das Kunststofformteil (2) eine
sternförmige Fläche mit jeweils um 120 versetzten Lappenteilen aufweist, die jeweils ein Kontaktteil ( 3 bis 5)
tragen, die etwa quadratisch ausgebildet sind und abgerundete Ecken haben.

6. Elektrodenanordnung nach Anspruch 1, d a d u r c h
g e k e n n z e i c h n e t , daß das Formteil (10)
aus isolierendem Kunststoff bandförmig ausgebildet ist,
in dem die Kontaktteile (11 bis 13) hintereinander angeordnet sind, die etwa dreiecksförmige Flächen aufweisen und mit ihren Flächenschwerpunkten gegeneinander
versetzt sind.

7. Elektrodenanordnung nach Anspruch 1, d a d u r c h
g e k e n n z e i c h n e t , daß die Anschlußleitungen (21 bis 23) der Kontaktteile (3 bis 5, 11 bis 13)
mit Teflonummantelungen schleifenförmig im Formteil (2,
10) einvulkanisiert sind.

8. Elektrodenanordnung nach Anspruch 1, d a d u r c h
g e k e n n z e i c h n e t , daß zum Anschluß des
Betriebskabels (7) am Formteil (2, 10) ein Kupplungsteil (24) bzw. Steckerteil angeordnet ist.

9. Anordnung nach Anspruch 1, d a d u r c h   g e -
k e n n z e i c h n e t , daß der Träger für das
Kontaktierungsmittel ein Vliesstoffteil (42) ist, dessen
Kontur (43) geringfügig größer als der äußere Umfang des
Formteiles (2,10) der Elektrodenanordnung (1,9) ist, wobei
das Vliesstoffteil (42) auf seiner der Applikationsfläche
abgewandten Seite eine Abdeckung (43) aus Isoliermaterial
aufweist, so daß die Elektrodenanordnung (1,9) zwischen
Vliesstoffteil (42) und Abdeckung (43) einschiebbar ist.

0029245

- 15 -    VPA 79 P 5116 EUR

10. Anordnung nach Anspruch 9, d a d u r c h   g e -
k e n n z e i c h n e t ,   daß das Vliesstoffteil (42)
ein Wirrfaservliesstoff (47) auf Zellwollbasis mit Polyolefinfasern ist, wobei wenigstens eine Seite des Wirrfaservliesstoffes (47) vollständig glatt ausgebildet ist.

11. Anordnung nach den Ansprüchen 9 und 10, d a -
d u r c h   g e k e n n z e i c h n e t ,   daß die
Kontur (48) des Vliesstoffteils (42) aus dem Wirrfaservliesstoff (47) in Spiegelsymmetrie doppelt gestanzt
ist, wobei durch Zusammenfalten längs der Spiegelsymmetrieebene (49) beide Oberflächen des Wirrfaservliesstoffteils (42) vollständig glatt sind.

12. Vorrichtung nach Anspruch 9, d a d u r c h
g e k e n n z e i c h n e t ,   daß das Vliesstoffteil
(42) mit der Abdeckung (43) bis auf eine Kante am Umfang
zusammengeheftet ist, so daß eine Tasche (41) für die
Elektrodenanordnung (1, 9) gebildet wird.

13. Vorrichtung nach Anspruch 9, d a d u r c h
g e k e n n z e i c h n e t ,   daß die Abdeckung
(43) aus transparenter Folie, vorzugsweise auf Polyvinyl-
chlorid-Basis, gebildet ist.

FIG 2

FIG 1

FIG 4

FIG 3

FIG 5

0029245

FIG 7

FIG 6

FIG 8

0029245

79 P 5116

FIG 9

Europäisches
Patentamt

## EUROPÄISCHER RECHERCHENBERICHT

| | EINSCHLÄGIGE DOKUMENTE | | KLASSIFIKATION DER ANMELDUNG (Int. Cl.) |
|---|---|---|---|
| Kategorie | Kennzeichnung des Dokuments mit Angabe, soweit erforderlich, der maßgeblichen Teile | betrifft Anspruch | |
| | <u>EP - A - 0 000 759</u> (SIEMENS)<br>* Seite 4, Zeilen 6-15; Seite 4, Zeile 32 bis Seite 5, Zeile 8; Seite 6, Zeilen 5-12; Seite 6, Zeile 24 bis Seite 7, Zeile 4; Seite 7, Zeilen 23-28; Seite 10, Zeile 12; Figuren 1-3 * | 1,2,4 | A 61 N 1/04<br>A 61 B 5/04 |
| | <u>FR - A - 1 098 726</u> (DUFLOT)<br>* Seite 2, Absätze 4,6,8,10; Zusammenfassung, Punkte 1,3,4 * | 1,7,9 | |
| | | | RECHERCHIERTE SACHGEBIETE (Int. Cl.) |
| | <u>US - A - 4 082 087</u> (HOWSON)<br>* Spalte 3, Zeilen 46-64; Spalte 4, Zeilen 27-31; Spalte 6, Zeilen 37-40; Figuren 2,3 * | 1,3,<br>6,7 | A 61 N 1/04<br>5/04<br>1/32 |
| | <u>FR - A - 787 477</u> (GOODRICH)<br>* Seite 2, Zeilen 80-104; Seite 3, Zeilen 1-45 * | 1,2,8 | |
| | <u>DE - A - 2 818 372</u> (SIEMENS)<br>* Seite 2, letzter Absatz; Seite 3; Figur 1 * | 2,7 | KATEGORIE DER GENANNTEN DOKUMENTE |
| | | | X: von besonderer Bedeutung<br>A: technologischer Hintergrund<br>O: nichtschriftliche Offenbarung<br>P: Zwischenliteratur |
| | <u>DE - A - 2 209 490</u> (WOHLMUTH)<br>* Seite 5, letzter Absatz; Seite 6, Absatz 1 * | 2,3 | T: der Erfindung zugrunde liegende Theorien oder Grundsätze<br>E: kollidierende Anmeldung<br>D: in der Anmeldung angeführtes Dokument<br>L: aus andern Gründen angeführtes Dokument |
| | <u>US - A - 3 960 413</u> (GLASSER) | 3 | &: Mitglied der gleichen Patentfamilie, übereinstimmendes Dokument |

Der vorliegende Recherchenbericht wurde für alle Patentansprüche erstellt.

| Recherchenort | Abschlußdatum der Recherche | Prüfer |
|---|---|---|
| Den Haag | 05-Dez-1981 | |

# EUROPÄISCHER RECHERCHENBERICHT

| | EINSCHLÄGIGE DOKUMENTE | | KLASSIFIKATION DER ANMELDUNG (Int. Cl.³) |
|---|---|---|---|
| Kategorie | Kennzeichnung des Dokuments mit Angabe, soweit erforderlich, der Maßgeblichen Teile | betrifft Anspruch | |
| | * Spalte 2, Zeilen 58-64; Spalte 4, Zeilen 5-16 *<br><br>-- | | |
| | <u>FR - A - 2 387 048</u> (PIAUBERT)<br> * Seite 2, Zeilen 21-30; Seite 4, Zeilen 2-17; Figur 2 *<br><br>-- | 5,9, 12 | |
| | <u>US - A - 2 590 876</u> (LANDAUER)<br> * Spalte 2, Zeilen 24-27, 39-46 und 52-55; Figur 1 *<br><br>-- | 6,8 | RECHERCHIERTE SACHGEBIETE (Int. Cl.³) |
| | <u>FR - A - 2 403 084</u> (CEA)<br> * Seite 3, Zeilen 15-17; Seite 4, Zeilen 31-33; Seite 5, Zeilen 24-34; Figur 1 *<br><br>-- | 7,8 | |
| | <u>FR - E - 0 072 894</u> (DARLET)<br> * Seite 1, linke Spalte; Figur 1 *<br><br>-- | 9,12 | |
| A | <u>DE - A - 2 411 389</u> (DEUTSCHE NEMECTRON)<br><br>----- | 1 | |